# FASCICULE DE BREVET EUROPEEN

(11) **EP 0 805 676 B1**
(45) Date de publication et mention de la délivrance du brevet: **15.12.1999**
(21) Numéro de dépôt: 96901859.7
(22) Date de dépôt: 26.01.1996
(51) Int. Cl.: A61K 9/20

(54) **COMPOSITIONS PHARMACEUTIQUES SOUS FORME DE COMPRIMES A LIBERATION PROLONGEE A BASE DE GRANULES EN POLYSACCHARIDES DE HAUT POIDS MOLECULAIRE**
ARZNEIMITTEL IN TABLETTENFORM MIT VERZÖGERTER WIRKSTOFFABGABE AUF BASIS VON HOCHMOLEKULAREN POLYSACCHARIDGRANULATEN
PHARMACEUTICAL COMPOSITIONS IN THE FORM OF SLOW RELEASE TABLETS BASED ON HIGH MOLECULAR WEIGHT POLYSACCHARIDE GRANULES

(30) Priorité: 27.01.1995 FR 9500946
(43) Date de publication de la demande: 12.11.1997
(73) Titulaire: RHODIA CHIMIE, 92408 Courbevoie Cédex (FR)
(72) Inventeur: COLLAUERI, Jean-Pierre, F-77410 Bordeux-par-Villevaude (FR); CONRATH, Guillaume, F-92290 Chatenay-Malabry (FR); DERIAN, Paul-Jo[l, F-92260 Fontenay-aux-Roses (FR); GOUSSET, Gabriel, F-92350 Plessis-Robinson (FR); MAUGER , Frédéric, F-42310 Changy (FR)
(74) Mandataire: Dutruc-Rosset, Marie-Claude
(86) Numéro de dépôt international: FR9600133
(87) Numéro de publication internationale: WO9622767

(56) Documents cités:
- EP-A- 0 206 368
- EP-A- 0 234 670
- EP-A- 0 360 562
- WO-A-87/05212
- WO-A-93/01803
- WO-A-94/15643
- FR-A- 2 600 267

## Description

La présente invention concerne des compositions pharmaceutiques sous forme de comprimés à libération prolongée (dénommées également à effet retard) et leur procédé de préparation.

Plus particulièrement, la présente invention vise des compositions du type ci-dessus dont les comprimés sont à base de granulés de polysaccharides de haut poids moléculaire, plus particulièrement à base de granulés de gomme xanthane.

Les compositions pharmaceutiques du type ci-dessus contiennent un principe pharmacologiquement actif et sont utilisés quand on souhaite administrer un médicament à un patient pendant une durée prolongée sans exiger du patient de prendre des doses répétées à intervalles réduits.

La compression directe est un procédé de galénique particulièrement intéressant car il fait intervenir un nombre limité d'opérations et de constituants et par conséquent nécessite pour sa mise en oeuvre des installations moins onéreuses que pour un procédé de préparation de comprimés par granulation humide. Cependant toutes les compositions pharmaceutiques ne peuvent pas être obtenues par cette voie.

Les gommes hydrophiles naturelles ou synthétiques qui sont des polysaccharides de haut poids moléculaire sont connues en tant qu'excipients pharmaceutiques mais toutes les gommes ne sont pas utilisables dans des compositions à effet prolongé dans les procédés de compression à sec pour préparer des comprimés. Ces polysaccharides sont soit d'origine microbienne et sont alors obtenus par fermentation d'un hydrate de carbone assimilable par un microorganisme approprié (exemple la gomme xanthane obtenue à partir de *Xanthomonas campestris*), soit d'origine naturelle comme par exemple les gommes guar et caroube.

Les gommes xanthanes sont également des excipients connus pour leur utilisation possible dans le domaine pharmaceutique, notamment pour la constitution de matrices destinées à la préparation de formes à libération contrôlée. Cependant les gommes hydrophiles en général et la gomme xanthane en particulier ne sont pas utilisés à l'état prégranulé.

EP-A-0 360 562 décrit des excipients à dissolution retardée, utilisables pour des compositions pharmaceutiques. Ces excipients comprennent 20 à 60 % d'un hétéropolysaccharide et un polysaccharide pouvant s'associer avec l'hétéropolysaccharide en présence d'eau, et de 40 à 80 % d'une charge.

WO-A-94/15643 a pour objet des compositions pharmaceutiques dont au moins 7% sont constitués par un glucomannane konjac comme agent de libération prolongé de la matière active.

Dans WO-A-93/01803, on décrit des médicaments à libération contrôlée de Verapamil dans l'appareil gastro-intestinal. L'excipient est constitué de gomme hydrophile comprenant un mélange de gomme xanthane et de gomme de caroube, ainsi qu'un diluant inerte comme les monosaccharides.

WO-A 87/05212 enseigne la préparation de comprimés à effet prolongé comportant une matrice formée de polysaccharides d'origine naturelle dont la gomme xanthane.

EP-A 234 670 enseigne également la préparation de comprimés à effet prolongé dans lesquels la matrice constitue 7,5 à 28 % en poids du comprimé, ladite matrice comportant de préférence au moins 75 % en poids de gomme xanthane.

Toutefois, l'utilisation de gomme xanthane non prégranulée, surtout à forte teneur c'est-à-dire généralement à une teneur d'environ 30 % en poids par rapport au poids total du comprimé, pour préparer un comprimé par compression directe, conduit à un comprimé présentant de nombreux défauts tels que clivages, hétérogénéité, mauvaises propriétés mécaniques, etc...

Un but de la présente invention est de préparer un comprimé à effet prolongé à base de polysaccharide de haut poids moléculaire, en particulier à base de gomme xanthane, pouvant être préparé sans difficulté par compression directe et présentant de bonnes propriétés de libération du principe actif et de bonnes propriétés mécaniques.

Ce but et d'autres sont atteints par la présente invention qui concerne en effet une composition pharmaceutique sous forme de comprimés à effet retard préparés par compression directe et constitués d'au moins un principe actif et d'une matrice conférant à ladite composition son effet retard, caractérisée en ce que ladite matrice est constituée au moins en partie de prégranulés de polysaccharides de haut poids moléculaire.

Les polysaccharides de haut poids moléculaire utilisables dans le cadre de la présente invention incluent les gommes hydrophiles qui peuvent être d'origine synthétique ou naturelle. Les polysaccharides de haut poids moléculaire d'origine synthétique, sont obtenus par fermentation d'un hydrate de carbone en présence de microorganismes.
Comme polysaccharides naturels ou naturels modifiés, on recommande d'utiliser les galactomanannes, les glucomanannes, les succinoglycanes,les scléroglucanes, les alginates, les carraghénanes, les gommes de caroube, de guar, de cassia et de tara, l'amidon, les dérivés de l'amidon, les pectines, le chitosan, et leurs diverses mélanges possibles. On peut utiliser des prégranulés d'un seul polysaccharide ou d'un mélange de polysaccharide. La gomme xanthane est le polysaccharide préféré. Par polysaccharides modifiés, on entend selon l'invention plus particulièrement leurs dérivés chimiques tels que par exemple les dérivés hydroxypropylés ou les dérivés carboxyméthylés des gommes.
On recommande plus particulièrement la gomme xanthane dont la préparation est décrite dans de nombreuses publications telles que US-A 3 020 206, US-A 3 391 060 et US-A 4 154 654.

On recommande également selon l'invention d'utiliser un autre type de polysaccharides présentant des propriétés épaississantes et de rhéologie adaptées aux mêmes types d'applications que celles de la gomme xanthane, constitué par les succcinoglycanes dont le motif de base contient du glucose, du galactose et un reste succinyle ; ils sont décrits dans les demandes de brevet européen EP-A 351 303 et EP-A 40 445, ainsi que dans Carbohydrate Research, 73 (1979) pp. 159-168, de Clarence A. Knutson ; ils peuvent être obtenus par fermentation microbienne d'un milieu comportant une source de carbone, au moyen d'un microorganisme appartenant au genre *Arthrobacter*, tel que *Arthobacter stabilis*, en particulier la souche *Arthobacter stabilis* NRRL-B-1973, au genre *Agrobacterium*, tels *Agrobacterium tumefaciens*, *Agrobacterium radiobacter* ou *Agrobacterium rhizogenes*, au genre *Rhizobium*, en particulier *Rhizobium meliloti* et *Rhizobium trifoli*, au genre *Alcaligenes*, tel *Alcaligenes faecalis*, en particulier la variété *myxogenes* ou au genre *Pseudomonas*, en particulier les souches *Pseudomonas sp*. NCIB 11264 et NCIB 11592. Parmi ces succinoglycanes, on peut tout particulièrement citer les gommes Rhéozan®, décrites dans la demande de brevet européen EP-A 351 303, et obtenues par fermentation d'une source carbonée au moyen de la souche *Agrobacterium tumefaciens* 1-736 déposée à la Collection Nationale de Culture des Microorganismes (CNCM).

Comme exemples de polysaccharides du commerce utilisables également dans le cadre de la présente invention, on peut citer le Wellan®, le Chamzan®, le Gellan®, le Dextran®, le Pullulan® et le Curdlan®.

Les prégranulés entrant dans la composition pharmaceutique selon l'invention sont avantageusement préparés en partant d'une poudre de polysaccharide, de préférence, de gomme Xanthane ayant une granulométrie telle que 90 % des particules ont une taille inférieure à 100 µm, de préférence, inférieure à 75 µm.
Le diamètre moyen est avantageusement compris entre 30 et 60 µm. On entend par diamètre moyen, un diamètre tel que 50 % en poids des particules ont un diamètre inférieur ou égal à ce diamètre.

Il existe de nombreux procédés décrits dans la littérature pour préparer des granulés de polysaccharide à partir de poudre de polysaccharide ou de solution de fermentation de polysaccharide. Dans le cadre de la présente invention tout procédé de granulation est utilisable, tel qu'atomisation, lit fluidisé, extrusion, granulation par plateaux tournants, etc....., ou combinaison de ces procédés.

Parmi ces procédés connus, on peut citer US-A 3 551 133 décrivant un procédé de préparation de cogranulés de gommes xanthane et de caroube à partir de la pulvérisation d'une solution aqueuse d'un mélange de poudre des deux gommes sur des granulateurs à plateau incliné ou à disque.

GB-A 2 086 204 décrit un procédé du même type.
Selon EP-A 206 368 on pulvérise une solution de gommes sur un lit fluidisé de gomme. US-A 4 557 938 décrit la préparation, au moyen d'un lit fluidisé, de granulés qui sont formés d'un mélange d'une gomme xanthane, guar ou caroube avec de l'amidon.

Le procédé préféré de granulation est, selon l'invention, le procédé selon lequel on pulvérise de la poudre de polysaccharide, de préférence de la gomme xanthane, en lit fluidisé à l'aide d'un courant gazeux et on pulvérise sur la poudre, de l'eau contenant éventuellement un tensio-actif et on obtient les granulés par séchage. Un tel procédé est décrit en détail dans FR-A 2 600 267. Selon une variante préférée, tous les excipients de la matrice sont cogranulés avant d'être comprimés.

On recommande d'utiliser pour préparer les compositions pharmaceutiques de l'invention, des prégranulés dont la granulométrie moyenne est comprise entre 50 µm et 1 000 µm, de préférence entre 100 et 350 µm et une densité apparente comprise entre 0,3 et 0,8 de préférence entre 0,35 et 0,7. La polysaccharide peut être le seul constituant de la matrice.

Cependant la matrice peut comporter en outre un ou plusieurs excipients pharmaceutiquement acceptables, plus particulièrement des agents diluants, des agents de cohésion, des agents lubrifiants et des agents colorants tels que notamment les saccharides, tels que le lactose et le sucrose, les acides gras tels que par exemple, l'acide stéarique, le polyéthylèneglycol, le phosphate dicalcique, la silice, les silicoaluminates, les dérivés cellulosiques, la gélatine, la polyvinylpyrrolidone et les sels d'acides gras comme le stéarate de magnésium.

Le polysaccharide, et plus particulièrement la gomme xanthane, forme en général entre 10 et 100 % en poids de la matrice, les autres excipients étant de préférence choisis parmi le lactose et le phosphate dicalcique. De préférence, la matrice constitue de 5 à 99,999 % en poids de la composition pharmaceutique.
Selon un mode de réalisation particulier de l'invention, la quantité de polysaccharide dans la matrice est d'au moins 20 % en poids total de ladite matrice.
Selon un autre mode de réalisation particulier de l'invention, la quantité de polysaccharide entrant dans la composition de la matrice est d'au plus 50 % en poids et de préférence d'au plus 40 % en poids.

Le lactose et/ou le phosphate dicalcique sont les co-excipients préférés. Ces excipients peuvent être co-granulés, le cas échéant, avec le ou les polysaccharides.

L'homme du métier n'aura dans ce cas aucune difficulté à faire varier le rapport lactose/phosphate pour obtenir la libération du principe actif sur la période de temps désirée, sachant que le lactose facilite notamment la libération et que l'effet du phosphate est plutôt inverse mais par ailleurs, il permet d'augmenter la densité des comprimés.

On peut selon l'invention faire varier très largement la proportion des différents ingrédients. Ainsi, on peut moduler la libération du principe actif d'une manière très importante et l'on peut passer d'une libération quasi instantanée à une libération sur 24 heures et plus. Généralement on souhaite une libération à effet retard afin de diminuer le nombre de prises quotidiennes, mais l'on ne sortira pas du cadre de l'invention, à préparer une composition pharmaceutique à libération rapide dès lors que l'on mettra en oeuvre dans cette demière, des prégranulés de polysaccharide.

On note aussi que les effets susmentionnés des deux co-excipients préférés dépendent de la quantité de polysaccharide présente dans la matrice. Ainsi, lorsque celle-ci est d'au moins 30 %, l'influence du lactose et du phosphate dicalcique est moins marquée.

Par exemple, lorsque l'on souhaite obtenir une composition pharmaceutique à libération rapide de la matière active, outre le polysaccharide, la matrice comprendra plus particulièrement une proportion plus élevée en lactose.
Par contre, si l'on souhaite obtenir une composition pharmaceutique à libération lente, c'est-à-dire au moins 12 heures, la matrice comprendra, outre le polysaccharide, une proportion plus importante de phosphate dicalcique.

La cinétique de libération dépend également de la nature de la matière active et notamment de sa plus ou moins grande solubilité dans l'eau.

La teneur en matière active entrant dans les compositions pharmaceutiques de l'invention peut varier dans de larges limites. Elle est plus particulièrement comprise entre 0,001 et 95 % en poids de la composition totale, le complément étant assuré par la matrice. Elle est préférentiellement comprise entre 0,01 et 12 % en poids. Selon un mode de réalisation préféré, elle est avantageusement comprise entre 0,1 et 10 % en poids.

La présente invention peut être utilisée pour la compression directe de principes actifs appartenant à toutes les classes de médicaments destinés à l'administration par voie orale. Parmi les principes actifs utilisés dans des compositions selon la présente invention on peut citer à titre non limitatif les anti-rhumatismaux et anti-inflammatoires non stéroïdiens (kétoprofène, ibuprofène, flurbiprofène, indométacine, phénylbutazone, allopurinol, nabumétone...), les analgésiques opiacés ou non (paracétamol, phénacétine, aspirine ...), les antitussifs (codéine, codéthyline, alimémazine ...), les psychotropes (trimipramine, amineptine, chlorpromazine et dérivés des phénothiazines, diazépam, lorazépam, nitrazépam, méprobamate, zopiclone, et dérivés de la famille des cyclopyrrolones ...), les stéroïdes (hydrocortisone, cortisone, progestérone, testostérone, prednisolone, triamcinolone, dexaméthazone, betaméthazone, paraméthazone, fluocinolone, béclométhazone ...), les barbituriques (barbital, allobarbital, phénobarbital, pentobarbital, amobarbital ...), les antimicrobiens (péfloxacine, sparfloxacine, et dérivés de la classe des quinolones, tétracylines, synergistines, métronidazole ...), les médicaments destinés au traitement des allergies, les anti-asthmatiques, les vitamines (vitamine A, vitamine E, vitamine du groupe D, vitamine K), les antispasmodiques et antisécrétoires (oméprazole), les cardiovasculaires et les vasodilatateurs cérébraux (quinacaïnol, oxprénolol, propanolol, nicergoline.....), les protecteurs cérébraux, les protecteurs hépatiques les agents thérapeutiques du tractus gastro-intestinal, les contraceptifs, les vaccins, les antihypertenseurs et les cardioprotecteurs tels que les béta-bloquants et les dérivés nitrés.

Grâce à l'utilisation de polysaccharide sous forme de prégranulés, tous les ingrédients constitutifs de la composition pharmaceutique peuvent être comprimés à sec par compression directe, c'est-à-dire sans utilisation d'un solvant organique tel que l'éthanol ou son mélange avec de l'eau.

Selon l'invention l'opération de compression consécutive au mélange des excipients et du principe actif est généralement mise en oeuvre sous une force pouvant aller de 6 à 10 kN (mesure au niveau du galet de compression) et de préférence de l'ordre de 8 à 9 kN. Celle opération de compression est, de préférence, précédée d'une pré-compression sous une force pouvant aller de 0,5 à 2,5 kN.

De grandes vitesses de compression peuvent être atteintes grâce au procédé selon l'invention, sans pour autant altérer la qualité des comprimés. Il est notamment possible d'atteindre des vitesses supérieures à 150 000 comprimés/heure, sans entraîner de clivage.

Il est entendu que les comprimés obtenus selon l'invention peuvent éventuellement être pelliculés selon les méthodes habituelles. L'opération de pelliculage est facilitée par le fait qu'aucun clivage n'intervient au cours de l'opération.

Les comprimés obtenus selon l'invention présentent l'avantage de pouvoir moduler la libération du principe acitf mais aussi de bonnes propriétés mécaniques, notamment la friabilité est inférieure à 1 %.

Le fait de partir d'un polysaccharide prégranulé, de préférence la gomme xanthane, présente également de nombreux avantages au niveau de la mise en oeuvre.
Ainsi, il présente des très bonnes propriétés d'écoulement ce qui favorise les étapes de transfert, de pesée, de mélange et de remplissage des chambres de compression. Par ailleurs, la granulométrie parfaitement contrôlée et l'absence de particules fines permettent de limiter les phénomènes de démélange et les risques liés aux pulvérulents fins. De plus, il possède de remarquables propriétés de comprimabilité ce qui facilité la préparation des comprimés.

Dans ce qui suit ou ce qui précède, sauf indications contraires, les pourcentages et parties sont en poids.
L'invention sera mieux comprise à la lecture des exemples suivants de réalisation donnés à titre illustratif nullement limitatif.

### EXEMPLES 1 A 18

### Mode opératoire de préparation de comprimés :

On mélange au préalable dans un mélangeur de type SONECO® 700 g du mélange de poudre constitué par le principe actif (2 g), en l'occurrence l'Aprikalim et les différents constituants de la matrice, à savoir la gomme xanthane prégranulée et éventuellement les autres excipients, à savoir le phosphate di-calcique et le lactose. Le mélange de poudre contient aussi l'agent lubrifiant (7 g) à savoir le stéarate de magnésium dosé donc à 1 % en poids dans ledit mélange de poudre.

La compression est mise en oeuvre sous une force de 8,5 kN (mesurée au niveau du galet de compression) en utilisant une machine rotative de type MANESTY® qui permet de réaliser, à partir de 700 g de mélange de poudre, 2 000 comprimés de 350 mg chacun, contenant donc 1 mg d'Aprikalim. Le mélange de poudre subit en outre une pré-compression sous une force de 1,5 kN.

On utilise de la gomme xanthane prégranulée (Rhodigel Easy® commercialisée par Rhône-Poulenc) dont 95 % en poids des granulés ont une granulométrie comprise entre 150 et 250 µm et une densité apparente de 0,5 g/cm3, préparée selon la technique du lit fluidisé décrite notamment dans le brevet français FR-A 2 600 267.

Les matrices des exemples 1 à 18 présentent différentes teneurs en excipients qui sont rassemblées dans le tableau 1 ci-après.

Sur les comprimés préparés, on apprécie les paramètres suivants dont les valeurs sont rassemblées dans le tableau 1 ci-après :
a) aspect macroscopique de comprimés et mise en évidence des clivages :
   - -: : pas de clivage,
   - +: : quelques clivages,
   - ++: : clivages significatifs,
b) la densité des comprimés mesurée au pycnomètre à hélium (résultat moyen des mesures effectuées sur 15 comprimés).
c) l'angle de mouillage (en degrés) et la vitesse d'absorption d'une goutte HCl 0,1 N (représentatif d'un milieu gastrique) mesurés au goniomètre et permettant d'évaluer la capacité d'hydratation instantanée du comprimé.
d) le coefficient de variation de poids (C.V. poids) des comprimés obtenus, mesure effectuée sur 40 comprimés.
e) le coefficient de variation de dureté des comprimés obtenus (C.V. dureté), mesure effectuée sur 15 comprimés et qui constitue un indicateur du clivage lors de la compression.
f) la cinétique de libération du principe actif dans les conditions de dissolution "sink" (volume du milieu de dissolution considéré comme infini) et en milieu présentant 2 pH différents, à savoir pH = 1,1 pendant 2 heures suivi de pH = 6,8 pendant 8 à 23 heures. Le principe actif est dosé par HPLC et détecteur UV. A partir de ces dosages, on évalue le profil de libération en fonction du temps et la durée nécessaire pour libérer 50 % en poids de l'Aprikalim.

Il ressort clairement du tableau 1 que les coefficients de variation en poids sont faibles, ce qui indique que les comprimés obtenus sur la machine rotative sont homogènes.
Il apparait en outre que lorsque la teneur en gomme xanthane augmente, cela diminue la mouillabilité instantanée du comprimé et limite la libération du principe actif. La forte potentialité de contrôle de la libération par la gomme xanthane est clairement mise en évidence par le tableau 1. Ainsi au temps 30 minutes, on passe de 7,5 % de libération à 65 % lorsque la teneur en gomme xanthane passe de 100 % à 12 %. La réalisation des exemples 5 et 6 permet de mettre en évidence la reproductibilité des profils de dissolution lorsqu'on fabrique deux lots selon la même composition. D' une manière générale, il est observé une absence totale de clivage ou une très faible tendance au clivage.

### EXEMPLE COMPARATIF

On répète exactement le mode opératoire des exemples 10 et 17 sauf que les granulés de gomme xanthane sont remplacés par de la gomme xanthane standard non prégranulée (Rhodigel® commercialisé par Rhône Poulenc) présentant une granulométrie centrée sur 100 µm, contenant 40 % de fines ayant une granulométrie comprise entre 35 et 50 µm. Les mélanges de poudre présentent une mauvaise comprimabilité.

En outre, les essais de fabrication de comprimés n'ont pas été concluants, les comprimés obtenus n'ayant pas une cohésion minimale et présentant un phénomène de clivage trop important.

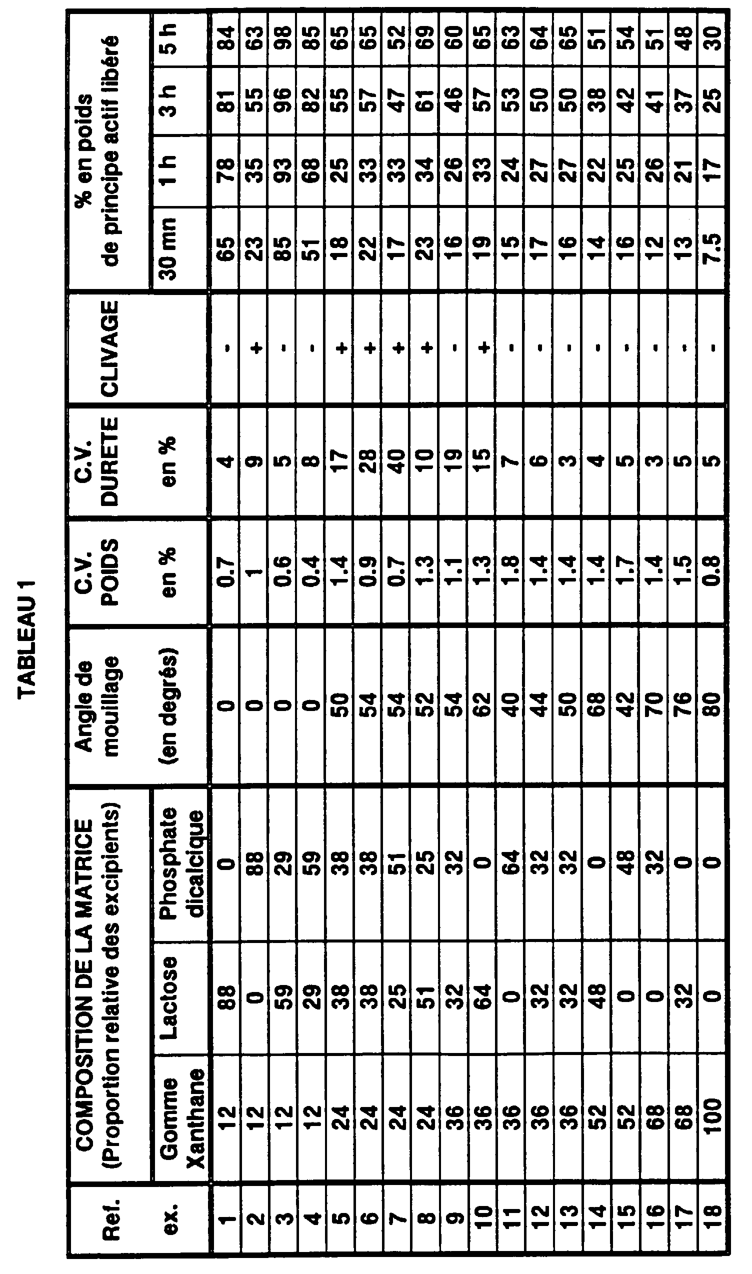

## Revendications

1. Composition pharmaceutique à effet retard se présentant sous forme de comprimés préparés par compression directe et constitués d'au moins un principe actif et d'une matrice conférant aux dites compositions son effet retard, caractérisée en ce que ladite matrice est constituée au moins en partie de prégranulés de polysaccharides de haut poids moléculaire, d'origine synthétique ou naturelle.

2. Composition pharmaceutique selon la revendication 1, caractérisée en ce que le polysaccharide est choisi parmi les polysaccharides naturels ou naturels modifiés en particulier les galactomanannes, les glucomanannes, les succinoglycanes,les scléroglucanes, les alginates, les carraghénanes, lesgommes de caroube, de guar, de cassia et de tara, l'amidon, les dérivés de l'amidon, les pectines, le chitosan, et leurs diverses mélanges possibles.

3. Composition pharmaceutique selon la revendication 1 ou 2, caractérisée en ce que la granulométrie moyenne des granulés est comprise entre 50 et 1 000, de préférence entre 100 et 350 µm et une densité apparente comprise entre 0,3 et 0,8, de préférence entre 0,35 et 0,7.

4. Composition pharmaceutique selon l'une quelconque des revendications précédentes, caractérisée en ce que la matrice comporte en outre au moins un excipient pharmaceutiquement acceptable choisi parmi des agents diluants, des agents de cohésion, des agents lubrifiants et des agents colorants.

5. Composition pharmaceutique selon la revendication 4, caractérisée en ce que la matrice comporte en outre au moins un excipient pharmaceutiquement acceptable choisi parmi les saccharides, tels que le lactose et le sucrose, les acides stéariques, le polyéthyléneglycol, le phosphate dicalcique, la silice, les silicoaluminates, les dérivés cellulosiques, la gélatine, la polyvinylpyrrolidone et les acides gras comme le stéarate de magnésium.

6. Composition pharmaceutique selon l'une quelconque des revendications précédentes, caractérisée en ce que le polysaccharide est la gomme xanthane.

7. Composition pharmaceutique selon l'une quelconque des revendications précédentes, caractérisée en ce que la poudre de gomme xanthane qui est prégranulée a un diamètre moyen compris entre 30 et 60 µm.

8. Composition pharmaceutique selon l'une revendications 6 et 7, caractérisée en ce que la gomme xanthane prégranulée est préparée par la technique du lit fluidisé.

9. Composition pharmaceutique selon l'une quelconque des revendications 5 à 8, caractérisée en ce que l'excipient supplémentaire est choisi parmi le lactose et le phosphate dicalcique.

10. Composition pharmaceutique selon l'une quelconque des revendications précédentes, caractérisée en ce que le polysaccharide est la gomme xanthane et forme entre 10 et 100 % en poids de la matrice.

11. Composition pharmaceutique selon l'une quelconque des revendications caractérisée en ce que la quantité de polysaccharide dans la matrice est d'au moins 20 % en poids total de ladite matrice.

12. Composition pharmaceutique selon l'une quelconque des revendications caractérisée en ce que la quantité de polysaccharide dans la matrice est d'au plus 50 % en poids et de préférence d'au plus 40 % en poids.

13. Composition pharmaceutique selon l'une quelconque des revendications précédentes, caractérisée en ce que matrice constitue de 5 à 99,999 % en poids de la composition pharmaceutique.

14. Composition selon l'une quelconque des revendications précédentes, caractérisée en ce que la teneur en matière active est comprise entre 0,001 et 95 % en poids de la composition totale, le complément étant assuré par la matrice ; de préférence, comprise entre 0,01 et 12 % en poids et encore plus préférentiellement comprise entre 0,1 et 10% en poids.

15. Composition selon l'une quelconque des revendications précédentes, caractérisée en ce que le principe actif est chois parmi : les anti-rhumatismaux et anti-inflammatoires non stéroïdiens, les antitussifs, les analgésiques opiacés ou non, les psychotropes, les stéroïdes, les barbituriques, les antimicrobiens, les médicaments destinés au traitement des allergies, les anti-asthmatiques, les vitamines, les antispasmodiques et antisécrétoires, les cardiovasculaires et les vasodilatateurs cérébraux, les protecteurs cérébraux, les protecteurs hépatiques les agents thérapeutiques du tractus gastro-intestinal, les contraceptifs, les vaccins, les antihypertenseurs et les cardioprotecteurs.

16. Procédé de préparation d'une composition pharmaceutique à effet retard se présentant sous forme de comprimés constitués d'au moins un principe actif et d'une matrice conférant aux dites compositions son effet retard, caractérisé en ce qu'il consiste à effectuer la compression directe d'une composition comprenant au moins un principe actif et une matrice constituée au moins en partie de prégranulés de polysaccharides de haut poids moléculaire, d'origine synthétique ou naturelle, de préférence, de Gomme Xanthane.

17. Procédé selon la revendication 16 caractérisé en ce que les granulés ont une granulométrie moyenne comprise entre 50 et 1 000, de préférence entre 100 et 350 µm et une densité apparente comprise entre 0,3 et 0,8, de préférence entre 0,35 et 0,7.

## Claims

1. Delayed-release pharmaceutical composition which is in the form of tablets prepared by direct tableting and consisting of at least one active principle and a matrix which gives the said composition its delayed-release effect, characterized in that the said matrix consists at least in part of pregranulated polysaccharides of high molecular weight and of synthetic or natural origin.

2. Pharmaceutical composition according to claim 1, characterized in that the polysaccharide is chosen from natural or modified natural polysaccharides, especially galactomannans, glucomannans, succinoglycans, scleroglucans, alginates, carragheenans, carob gum, guar gum, cassia gum and tara gum, starch, starch derivatives, pectins, chitosan, and their various possible mixtures.

3. Pharmaceutical composition according to claim 1 or 2, characterized in that the average particle size of the granules is between 50 and 1000 µm, preferably between 100 and 350 µm, and their apparent density is between 0.3 and 0.8, preferably between 0.35 and 0.7.

4. Pharmaceutical composition according to any one of the preceding claims, characterized in that the matrix additionally comprises at least one pharmaceutically acceptable excipient chosen from diluents, cohesion agents, lubricants and colorants.

5. Pharmaceutical composition according to claim 4, characterized in that the matrix additionally comprises at least one pharmaceutically acceptable excipient chosen from saccharides, such as lactose and sucrose, stearic acids, polyethylene glycol, dicalcium phosphate, silica, silicoaluminates, cellulose derivatives, gelatin, polyvinylpyrrolidone and the salts of fatty acids, such as magnesium stearate.

6. Pharmaceutical composition according to any one of the preceding claims, characterized in that the polysaccharide is xanthan gum.

7. Pharmaceutical composition according to any one of the preceding claims, characterized in that the xanthan gum powder which is pregranulated has an average diameter of between 30 and 60 µm.

8. Pharmaceutical composition according to one of claims 6 and 7, characterized in that the pregranulated xanthan gum is prepared by the fluidized-bed technique.

9. Pharmaceutical composition according to any one of claims 5 to 8, characterized in that the additional excipient is chosen from lactose and dicalcium phosphate.

10. Pharmaceutical composition according to any one of the preceding claims, characterized in that the polysaccharide is xanthan gum and forms between 10 and 100 % by weight of the matrix.

11. Pharmaceutical composition according to any one of the preceding claims, characterized in that the amount of polysaccharide in the matrix is at least 20% by total weight of the said matrix.

12. Pharmaceutical composition according to any one of the preceding claims, characterized in that the amount of polysaccharide in the matrix is at most 50% by weight and preferably at most 40% by weight.

13. Pharmaceutical composition according to any one of the preceding claims, characterized in that the matrix makes up from 5 to 99.999 % by weight of the pharmaceutical composition.

14. Composition according to any one of the preceding claims, characterized in that the active substance content is between 0.001 and 95% by weight of the total composition, the remainder being provided by the matrix; preferably between 0.01 and 12% by weight and, still more preferably, between 0.1 and 10% by weight.

15. Composition according to any one of the preceding claims, characterized in that the active principle is selected from nonsteroidal anti-inflammatories and anti-rheumatics, antitussives, opiate or non-opiate analgesics, psychotropic agents, steroids, barbiturates, antimicrobial agents, medicaments intended for the treatment of allergies, antasthmatics, vitamins, antispasmodics and antisecretory agents, cardiovascular agents and cerebral vasodilators, cerebroprotective agents, hepatic protective agents, therapeutic agents of the gastrointestinal tract, contraceptives, vaccines, antihypertensives and cardioprotective agents.

16. Process for the preparation of a delayed-release pharmaceutical composition which is in the form of tablets consisting of at least one active principle and a matrix which gives the said composition its delayed-release effect, characterized in that it consists in carrying out the direct tableting of a composition comprising at least one active principle and a matrix consisting at least in part of pregranulated polysaccharides of high molecular weight and of synthetic or natural origin, preferably xanthan gum.

17. Process according to claim 16, characterized in that the granules have an average particle size of between 50 and 1000, preferably between 100 and 350 µm and an apparent density of between 0.3 and 0.8, preferably between 0.35 and 0.7.

## Patentansprüche

1. Pharmazeutische Zusammensetzung mit Retardwirkung in Form von Tabletten, die hergestellt sind durch direkte Tablettierung und die aus mindestens einem Wirkstoff und einer Matrix bestehen, welche diesen Zusammensetzungen ihre Retardwirkung verleiht, dadurch gekennzeichnet, dass die Matrix zumindest teilweise aus einem Vorgranulat aus Polysacchariden mit hohem Molekulargewicht synthetischen oder natürlichen Ursprungs besteht.

2. Pharmazeutische Zusammensetzung nach Anspruch 1, dadurch gekennzeichnet, dass das Polysaccharid ausgewählt ist aus natürlichen und modifizierten natürlichen Polysacchariden, insbesondere Galactomannanen, Glucomannanen, Succinoglycanen, Skleroglucanen, Alginaten, Carrhagenanen, Carubgummi, Guargummi, Cassiagummi und Taragummi, Stärke, Stärke-derivaten, Pektinen, Chitosan und deren verschiedenen, möglichen Mischungen.

3. Pharmazeutische Zusammensetzung nach Anspruch 1 oder 2, dadurch gekennzeichnet, dass das Granulat eine mittlere Teilchengröße zwischen 50 und 1.000 µm, vorzugsweise zwischen 100 und 350 µm, und eine Schüttdichte zwischen 0,3 und 0,8, vorzugsweise zwischen 0,35 und 0,7, aufweist.

4. Pharmazeutische Zusammensetzung nach einem der vorhergehenden Ansprüche, dadurch gekennzeichnet, dass die Matrix außerdem mindestens einen pharmazeutisch verträglichen Träger umfasst, ausgewählt aus Füllstoffen, Bindemitteln, Gleitmitteln und Farbstoffen.

5. Pharmazeutische Zusammensetzung nach Anspruch 4, dadurch gekennzeichnet, dass die Matrix außerdem mindestens einen pharmazeutisch verträglichen Träger umfasst, ausgewählt aus Sacchariden wie Lactose und Sucrose, Stearinsäuren, Polyethylenglykol, Calciumhydrogenphosphat, Siliciumdioxid, Siliciumaluminaten, Cellulosederivaten, Gelatine, Polyvinylpyrrolidon und Fettsäuren wie Magnesiumstearat.

6. Pharmazeutische Zusammensetzung nach einem der vorhergehenden Ansprüche, dadurch gekennzeichnet, dass das Polysaccharid Xanthangummi ist.

7. Pharmazeutische Zusammensetzung nach einem der vorhergehenden Ansprüche, dadurch gekennzeichnet, dass das Xanthangummipulver, welches vorgranuliert ist, einen mittleren Durchmesser zwischen 30 und 60 µm aufweist.

8. Pharmazeutische Zusammensetzung nach Anspruch 6 oder 7, dadurch gekennzeichnet, dass das vorgranulierte Xanthangummi mittels Fließbettverfahren hergestellt ist.

9. Pharmazeutische Zusammensetzung nach einem der Ansprüche 5 bis 8, dadurch gekennzeichnet, dass der zusätzliche Träger aus Lactose und Calciumhydrogenphoshat ausgewählt ist.

10. Pharmazeutische Zusammensetzung nach einem der vorhergehenden Ansprüche, dadurch gekennzeichnet, dass das Polysaccharid Xanthangummi ist und 10 bis 100 Gew.-% der Matrix bildet.

11. Pharmazeutische Zusammensetzung nach einem der vorhergehenden Ansprüche, dadurch gekennzeichnet, dass die Polysaccharidmenge in der Matrix mindestens 20 Gew.-% der gesamten Matrix beträgt.

12. Pharmazeutische Zusammensetzung nach einem der vorhergehenden Ansprüche, dadurch gekennzeichnet, dass die Polysaccharidmenge in der Matrix höchstens 50 Gew.-%, vorzugsweise höchstens 40 Gew.-%, beträgt.

13. Pharmazeutische Zusammensetzung nach einem der vorhergehenden Ansprüche, dadurch gekennzeichnet, dass die Matrix 5 bis 99,999 Gew.-% der pharmazeutischen Zusammensetzung darstellt.

14. Zusammensetzung nach einem der vorhergehenden Ansprüche, dadurch gekennzeichnet, dass der Wirkstoffgehalt 0,001 bis 95 Gew.-% der Gesamtzusammensetzung, vorzugsweise 0,01 bis 12 Gew.-%, insbesondere 0,1 bis 10 Gew.-%, beträgt, wobei der Rest auf die Matrix entfällt.

15. Zusammensetzung nach einem der vorhergehenden Ansprüche, dadurch gekennzeichnet, dass der Wirkstoff ausgewählt ist aus Antirheumatika und nichtsteroidalen antiinflammatorischen Mitteln, Antitussiva, Analgetika auf Opiatbasis oder Nichtopiatbasis, Psychopharmaka, Steroiden, Barbituraten, antimikrobiell wirkenden Substanzen, Medikamenten zur Behandlung von Allergien, Mitteln gegen Asthma, Vitaminen, Antispasmodika und schleimhemmenden Mitteln, cardiovaskulären und vasodilatorischen cerebral wirkenden Substanzen, cerebralen Protektoren, Mitteln zum Schutz der Leber, Mitteln zur Behandlung des Gastrointestinaltraktes, Kontrazeptiva, Impfstoffen, Antihypertensiva und Mitteln zum Schutz des Herzens.

16. Verfahren zur Herstellung einer pharmazeutischen Zusammensetzung mit Retardwirkung in Form von Tabletten, die aus mindestens einem Wirkstoff und einer Matrix bestehen, welche diesen Zusammensetzungen ihre Retardwirkung verleiht, dadurch gekennzeichnet, dass man eine Zusammensetzung, die mindestens einen Wirkstoff und eine Matrix umfaßt, welche zumindest teilweise aus einem Vorgranulat aus Polysacchariden mit hohem Molekulargewicht synthetischen oder natürlichen Ursprungs, vorzugsweise Xanthangummi, besteht, direkt tablettiert.

17. Verfahren nach Anspruch 16, dadurch gekennzeichnet, dass das Granulat eine mittlere Teilchengröße zwischen 50 und 1.000 µm, vorzugsweise zwischen 100 und 350 µm, und eine Schüttdichte zwischen 0,3 und 0,8, vorzugsweise zwischen 0,35 und 0,7, aufweist.
